# EUROPÄISCHE PATENTSCHRIFT

(11) **EP 2 250 962 B1**
(45) Veröffentlichungstag und Bekanntmachung des Hinweises auf die Patenterteilung: **31.07.2019**
(21) Anmeldenummer: 10158185.8
(22) Anmeldetag: 29.03.2010
(51) Int. Cl.: A61B 5/0452, A61N 1/00

(54) **Implantierbarer P-Wellen-Dispersionsdetektor**
Implantable P-wave dispersion detector
Détecteur de dispersion à ondes P implantable

(30) Priorität: 11.05.2009 DE 102009002988
(43) Veröffentlichungstag der Anmeldung: 17.11.2010
(73) Patentinhaber: BIOTRONIK SE & Co. KG, 12359 Berlin (DE)
(72) Erfinder: Dörr, Thomas, 12437, Berlin (DE)
(74) Vertreter: Keck, Hans-Georg

(56) Entgegenhaltungen:
- US-A1- 2002 123 769
- US-A1- 2006 173 369
- US-B1- 7 010 346

## Beschreibung

Die Erfindung betrifft ein elektromedizinisches Implantat, das zum Bestimmen einer P-Wellendispersion ausgebildet ist. Das elektromedizinische Implantat kann beispielsweise ein entsprechend konfigurierter implantierbarer Herzschrittmacher oder ein implantierbarer Kardioverter/Defibrillator (ICD) oder eine Kombination aus Beidem sein.

Wie derartige bekannte Geräte auch besitzt das elektromedizinische Implantat eine Aufnahmeeinheit zum Aufnehmen eines Elektrokardiogrammsignals. Diese Aufnahmeeinheit ist wenigstens mit zwei Elektroden zur Aufnahme elektrischer Signale verbunden oder zu verbinden, die den Verlauf eines Elektrokardiogrammsignals des rechten und des linken Vorhofes widerspiegeln.

Außerdem besitzt das elektromedizinische Implantat eine Detektionseinheit, die ausgebildet ist, für atriale Herzaktionen kennzeichnende Signalmerkmale in einem Elektrokardiogrammsignal zu detektieren. Letzteres geschieht bei bekannten implantierbaren Herzschrittmachern oder ICD dadurch, dass ein im Atrium eines Herzens aufgenommenes intraatriales Elektrokardiogrammsignal permanent einem Schwellwertvergleich unterzogen wird. Überschreitet die Amplitude des intraatrialen Elektrokardiogrammsignals den Schwellwert, wird eine atriale Herzaktion detektiert. Typischerweise ist hierfür eine atriale Sensing-Einheit vorgesehen. Die Detektion einer atrialen Herzaktion wird auch als atriales Sense-Ereignis bezeichnet. Das jeweils detektierte elektrische Signal ist Folge einer mit der Kontraktion des atrialen Myokards einhergehenden Dipolarisierung der atrialen Muskelzellen.

Die Erfassung und Auswertung der atrialen Dispersion ist ebenfalls grundsätzlich bekannt. Die P-Wellen-Dispersion beschreibt die zeitliche Verteilung der P-Welle in den 12-Ableitungen des Oberflächen-Elektrokardiogramm und ist somit Ausdruck der atrialen Erregungsausbreitung. Vergrößert sich der Wert der P-Wellendispersion ist von einer inhomogenen atrialen Erregungsbildung und -leitung auszugehen. Insbesondere die durch mehrere Fokusse initiierte atriale Erregung im Vorfeld einer atrialen Arrhythmie verursacht eine inhomogene Erregung des Vorhofs und somit eine größere P-Wellen-Dispersion.

Die P-Wellen-Dispersion stellt also eine wichtige diagnostische Information für Patienten mit einem Risiko für Vorhofarrhythmien dar. Da die Koinzidenz von Vorhofflimmern und die Indikation für einen ICD sehr hoch ist, hat dieser Parameter für diese Patienten eine große Bedeutung.

In US 2005/0027321 A1 wird eine Vorrichtung beschrieben, die unter anderem auch die Morphologie der P-Welle einschließlich deren Dauer bestimmen kann und auch die "dispersions of widths of the P-waves" aufzeichnen soll. Der Begriff der Dispersion wird in US 2005/0027321 A1 etwas abweichend verwendet: man meint hier damit den Vergleich der Varianz der P-Wellenbreite aufgezeichnet über mehrere aufeinanderfolgende P-Wellen. Weitere Beispiele für die Bestimmung der P-Wellen in elektronischen Implantaten sind in US 7010346 B1 und US 2002/123769 A1 offenbart.

Die in der Literatur beschriebene P-Wellendispersion ist jedoch definiert im 12-Kanal-Oberflächen-Elektrokardiogramm als eine zeitliche "Zerstreuung" einer P-Welle gemessen in den 12-Ableitungen des Oberflächen-Elektrokardiogramm. Diese P-Wellendispersion ist damit Ausdruck der atrialen Erregungsbildung und -ausbreitung. Genau dies soll mit der beschriebenen Erfindung in einem elektronischen Implantat realisiert werden, ohne dass dazu eine atriale Elektrode zwingend erforderlich ist.

Die Studie "Koide Y, Yotsukura M, Ando H, Aoki S, Suzuki T, Sakata K, Ootomo E, Yoshino H. ""Usefulness of P-wave dispersion in standard twelve-lead electrocardiography to predict transition from paroxysmal to persistent atrial fibrillation." Am J Cardiol. 2008 Sep 1;102(5):573-7. Epub 2008 Jul 10" berichtet von dem diagnostischen Werten der P-Wellen-Dispersion, abgeleitet aus dem 12-Kanal-Oberflächen-Elektrokardiogramm.

Bislang ist es nicht möglich, diesen Parameter in einem ICD automatisch zu erfassen.

Die Aufgabe der Erfindung besteht darin, einen der P-Wellen-Dispersion des 12-Kanal-Oberflächen-Elektrokardiogramms entsprechenden Parameter in einem implantierbaren Gerät (z. B. ICD, Herzschrittmacher, Rhythmusmonitor) zu erfassen und damit einen prediktiven diagnostischen Wert für das Management vom Vorhofflimmerpatienten bereitzustellen.

Wichtig ist dabei die exakte Bestimmung des frühesten Beginns der atrialen Erregung und des Endes der atrialen Erregung.

Erfindungsgemäß wird diese Aufgabe durch ein elektromedizinisches Implantat gelöst, das eine Fernfeld-Elektrokardiogrammerfassungseinheit zum Aufnehmen eines Fernfeld-Elektrokardiogrammsignals aufweist, die mit wenigstens zwei Elektroden zur Aufnahme solcher elektrischer Signale verbunden oder zu verbinden ist, die den Verlauf eines zur Aufnahme solcher elektrischer Signale verbunden oder zu verbinden ist, die den Verlauf eines Fernfeld-Elektrokardiogramms des rechten und des linken Vorhofs widerspiegeln. Außerdem weist das erfindungsgemäße elektromedizinische Implantat eine Detektionseinheit auf, die ausgebildet ist, für atriale Herzaktionen kennzeichnende Signalmerkmale in einem Elektrokardiogrammsignal zu detektieren. Weiterhin weist das elektromedizinische Implantat eine Mittelungseinheit auf, die mit der Aufnahmeeinheit und der Detektionseinheit verbunden und ausgebildet ist, ein gemitteltes P-Wellen-Signal zu generieren, in dem die Mittelungseinheit eine Mehrzahl von einer jeweiligen detektierten atrialen Herzaktion zugeordneten Signalabschnitten des Fernfeld-Elektrokardiogrammsignals miteinander mittelt. Schließlich besitzt das elektromedizinische Implantat eine Auswerteeinheit, die mit der Mittelungseinheit verbunden ist und ausgebildet ist, in dem jeweiligen gemittelten P-Wellen-Signal die Dauer einer gemittelten P-Welle zu bestimmen.

Die Dauer einer jeweiligen gemittelten P-Welle spiegelt dabei die Dispersion der P-Welle wider.

Die Erfindung beruht auf der Erkenntnis, dass ein mittels eines Implantats aufgenommenes Fernfeld-Elektrokardiogrammsignal, welches die Aktivität beider Vorhöfe widerspiegelt, besonders dafür geeignet ist, hinsichtlich einer P-Wellen-Dispersion analysiert zu werden.

Bei aus dem Stand der Technik bekannten Implantaten beruht die Analyse von P-Wellen typischerweise auf einem intraatrialen Elektrokardiogramm, welches mit einer relativ kleinflächigen Elektrode in einem Atrium eines Herzens aufgenommen wird. Derartige Elektrokardiogramme spiegeln regelmäßig im Wesentlichen die Aktivität eines einzigen Vorhofs, aber nicht beider Vorhöfe (Atrien) wider.

Der Vorteil der erfindungsgemäßen Lösung besteht darin, dass nunmehr die diagnostische Information der P-Wellen-Dispersion in einem elektronischen Implantat, wie z. B. einem Herzschrittmacher, ICD oder implantierbaren Monitor erfasst werden kann. Dabei können die für die üblichen Anwendungen eines solchen Gerätes bereits vorhandenen Elektroden eingesetzt werden.

Mit dieser diagnostische Information kann das Management von Vorhofflimmerpatienten erheblich verbessert werden, da nun ein kontinuierliches Monitoring der P-Wellendispersion möglich ist und so z. B. der Zeitpunkt einer Medikationsumstellung oder einer Vorhofflimmerablation vorausschauend geplant werden kann.

Diese zusätzliche atriale Diagnostik kann auch zu einem differenzierterem Einsatz der Vorhofflimmerablation beitragen und somit die z. Z. noch zu hohen Kosten der Ablation zu senken.

Im Folgenden werden nun einige vorteilhafte Ausgestaltungen näher erläutert.

Die Detektionseinheit zum Erfassen atrialer Herzaktionen kann eine übliche atriale Sensingeinheit sein, die mit einer üblichen atrialen Elektrode verbunden sein kann. Vorzugsweise ist jedoch die Detektionseinheit mit der Fernfeld-Elektrokardiogrammerfassungs-einheit verbunden und ausgebildet, atriale Herzaktionen, beispielsweise anhand von Morphologiekriterien oder anderen Kriterien, in dem von der Fernfeld-Elektrokardiogrammerfassungseinheit aufgenommenen Fernfeld-Elektrokardiogramm zu detektieren. Die letztgenannte Variante hat den Vorteil, dass sie eine Erfassung und Analyse der P-Wellen-Dispersion auch mit solchen Implantaten ermöglicht, die keine eigene atriale Elektrode aufweisen, also beispielsweise bei Einkammer-Herzschrittmachern oder Einkammer-ICDs.

Das der Detektionseinheit zugeführte Elektrokardiogramm ist vorzugsweise gefiltert. Hierzu weist das Implantat vorzugsweise insbesondere einen Bandpassfilter auf, der auf den Frequenzbereich der zu detektierenden Signale abgestimmt ist.

Zur Aufnahme eines Fernfeld-Elektrokardiogrammsignals ist die Fernfeld-Elektrokardiogrammerfassungseinheit vorzugsweise wenigstens mit einem elektrisch leitenden Teil eines Gehäuses des Implantats mittels einer ersten Elektrode verbunden. Dies erlaubt zum einen die Aufnahme eines Fernfeld-Elektrokardiogramms, da sich das Gehäuse des Implantats außerhalb des Herzens in einem geeigneten Abstand befindet. Außerdem macht es diese Variante überflüssig, eine gesonderte Elektrode zu implantieren.

Weiterhin ist es bevorzugt, wenn die Fernfeld-Elektrokardiogrammerfassungseinheit mit einer Defibrillationselektrode an einer entsprechenden, mit dem Implantat verbundenen Elektrodenleitung als zweite Elektrode verbunden ist. Defibrillationselektroden sind in der Regel als Schockwendeln ausgebildet und im Vergleich zu typischen Stimulations- oder Sensingelektroden relativ großflächig. Eine besonders geeignete Elektrode ist eine atriale Schockwendel. Durch eine großflächige Elektrode als zweite Elektrode zur Aufnahme eines Fernfeld-Elektrokardiogramms wird bewirkt, dass das aufgenommene Elektrokardiogramm tatsächlich ein Fernfeld-Elektrokardiogramm ist.

Vorzugsweise ist das Implantat ist ein ICD, verbunden mit den üblichen Elektroden.

Speziell in diesem Fall (aber auch bei anderen Implantaten) kann die Fernfeld-Elektrokardiogrammerfassungseinheit auch mit an sich bekannten atrialen oder ventrikulären Ring- oder Tipelektroden verbunden sein, beispielsweise sind folgende Konfigurationen möglich:
Die breitbandige Ableitung des Fernfeld-Elektrokardiogramms zur Bestimmung der P-Wellendispersion erfolgt zwischen einer distalen Schockelektrode und dem Gehäuse eines ICD.

Die breitbandige Ableitung des Fernfeld-Elektrokardiogramms zur Bestimmung der P-Wellendispersion erfolgt zwischen einer proximalen Schockelektrode und dem Gehäuse eines ICD.

Die breitbandige Ableitung des Fernfeld-Elektrokardiogramms zur Bestimmung der P-Wellendispersion erfolgt zwischen rechtsventrikulären Ringelektrode und dem Gehäuse eines ICD oder Herzschrittmachers.

Die breitbandige Ableitung des Fernfeld-Elektrokardiogramms zur Bestimmung der P-Wellendispersion erfolgt zwischen einer rechtsventrikulären Tipelektrode und dem Gehäuse eines ICD oder Herzschrittmachers.

Vorteilhaft ist es auch, wenn die für die breitbandige Ableitung des Fernfeld-Elektrokardiogramms zur Bestimmung der P-Wellendispersion genutzten Elektrodenkonfigurationen manuell umgeschaltet oder umprogrammiert werden können.

Alternativ kann auch vorgesehen sein, dass die jeweilige für die breitbandige Ableitung des Fernfeld-Elektrokardiogramms zur Bestimmung der P-Wellendispersion genutzte E-lektrodenkonfiguration basierend auf einer Elektrokardiogramm-Signalqualitätsprüfung automatisch ausgewählt wird.

Die Auswerteeinheit besitzt vorzugsweise sowohl eine Einheit zum Bestimmen eines Anfangszeitpunkts einer gemittelten P-Welle als auch eine Einheit zum Bestimmen eines Endzeitpunkts einer gemittelten P-Welle. Diese beiden Einheiten können auch separat von der Auswerteeinheit vorgesehen sein und mit dieser verbunden sein. Die Auswerteeinheit ist dazu ausgebildet, die Dauer einer gemittelten P-Welle als Zeitdifferenz zwischen dem von der einen Einheit ermittelten Anfangszeitpunkt und dem von der anderen Einheit ermittelten Endzeitpunkt zu bestimmen. Diese beiden Einheiten zum Bestimmen des Anfangszeitpunkts bzw. des Endzeitpunkts einer gemittelten P-Welle sind vorzugsweise so ausgebildet, dass für sie jeweils eigene Detektionsparameter, wie z. B. Signalverstärkungsfaktor, Schwellwerte, verwendete Signale etc., vorgegeben werden können. Zu den auszuwählenden Vorgaben kann es auch gehören, ein bestimmtes von mehreren Elektrokardiogrammsignalen hinsichtlich des jeweiligen Zeitpunkts auszuwerten.

Besonders vorteilhaft ist es, wenn die Mittelungseinheit, die Auswerteeinheit oder die Einheit zum Bestimmen eines Endzeitpunkts einer gemittelten P-Welle dazu ausgebildet ist, von einer jeweiligen P-Welle oder einem zu mittelnden Signalabschnitt ein zeitgleiches ventrikuläres Elektrokardiogramm abzuziehen, d.h. die Amplitude eines synchronen ventrikulären Elektrokardiogramms von der jeweils entsprechenden Amplitude der P-Welle oder des zu mittelnden Signalabschnitts zu subtrahieren.

Die Mittelungseinheit ist vorzugsweise dazu ausgebildet, miteinander zu mittelnde Signalabschnitte so miteinander zu mitteln, dass sie über die jeweiligen Zeitpunkte der Detektion einer atrialen Herzaktion miteinander synchronisiert sind. Der Zeitpunkt der Detektion einer jeweiligen atrialen Herzaktion ist somit für jeden der zu mittelnden Signalabschnitte der Bezugszeitpunkt für die Mittlung der Signalabschnitte miteinander.

In Bezug auf die Mittelungseinheit ist es außerdem vorzugsweise vorgesehen, dass die Anzahl aufeinander folgender zu mittelnder Signalabschnitte einstellbar ist.

Gemäß einer bevorzugten Ausführungsvariante weist das Implantat eine Monitoringeinheit auf, die einen der P-Wellen-Dispersion entsprechenden Parameter - also insbesondere die von der Auswerteeinheit ermittelte Dauer der gemittelten P-Wellen regelmäßig, und zwar vorzugsweise kontinuierlich oder mindestens einmal täglich, überwacht.

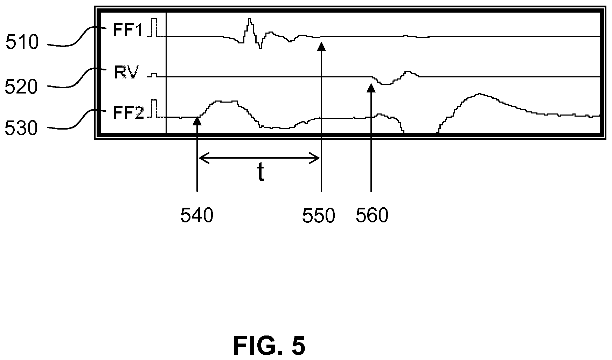

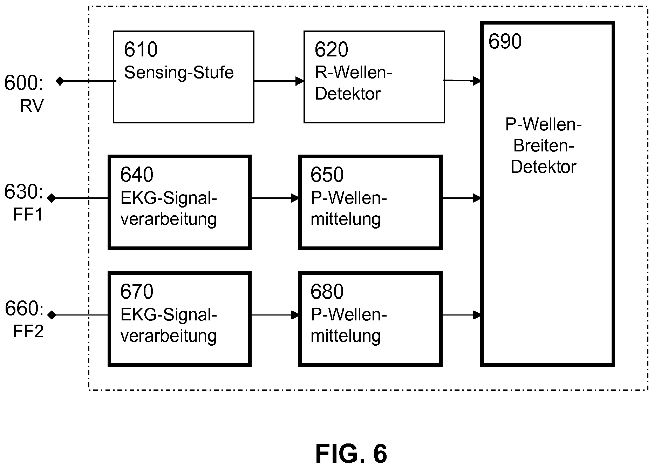

Besonders vorteilhaft ist es, wenn die Fernfeldelektrokardiogrammerfassungseinheit dazu ausgebildet ist, wenigstens zwei Fernfeldelektrokardiogrammsignale parallel aufzunehmen, wie dies nachfolgend mit Bezug auf die Figuren 5 und 6 näher erläutert ist.

Gemäß einer weiteren vorteilhaften Ausführungsvariante erfolgt eine breitbandige Ableitung des Fernfeld-Elektrokardiogramms zur Bestimmung der P-Wellendispersion über eine rechtsatriale, flottierende Elektrode (VDD-Prinzip).

Die Erfindung soll nun anhand eines Ausführungsbeispiels mit Bezug auf die Figuren näher erläutert werden. Von den Figuren zeigt:
- Fig. 1:: ein Einkammer-ICD-System als Beispiel für ein elektromedizinisches Implantat;
- Fig. 2:: eine beispielhafte Darstellung einer P-Welle in einem Fernfeld-Elektrokardiogramm;
- Fig. 3:: ein Blockschaltbild einiger Komponenten eines Implantats;
- Fig. 4:: Ein Beispiel für ein Implantat in einem Fernübertragungssystem;
- Fig. 5:: ein Beispiel für eine mehrkanalige P-Wellendauer-Bestimmung; und
- Fig. 6:: einen Mehrkanaldetektor zur P-Wellendauer-Bestimmung

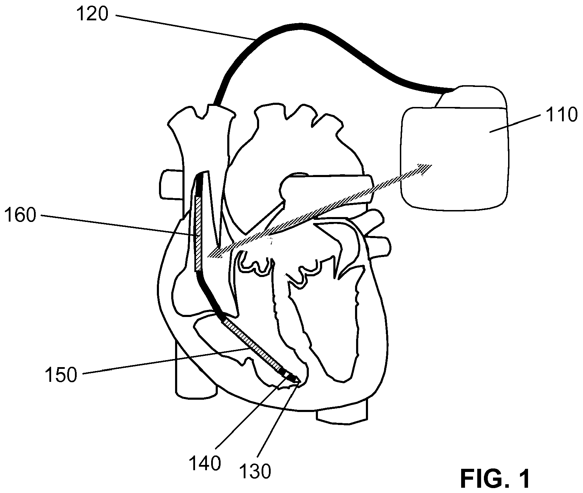

Als mögliches Ausführungsbeispiel ist in der Figur 1 ein Einkammer-ICD-System dargestellt. Dessen Gehäuse 110 samt der darin befindlichen Komponenten ist mit einer flexiblen, implantierbaren Elektrodenleitung 120 verbunden. Diese weist an ihrem distalen Ende einen bipolaren Wahrnehmungs- und Stimulationspol, bestehend aus einer rechtsventrikulären Tipelektrode 130 und einer rechtsventrikulären Ringelektrode 140 auf. Zur Defibrillationsschockabgabe sind an der Elektrodenleitung 120 eine distale Schockwendel 150 und optional eine proximale Schockwendel 160 als jeweilige Defibrillations- oder Schockelektrode angebracht.

Die für die erfindungsgemäße Bestimmung der P-Wellen-Disperion benötigten Elektrokardiogramme können prinzipiell von folgenden Elektrodenkombinationen abgeleitet werden:

| | |
|---|---|
| Variante A: | rechtsventrikulären Tipelektrode - ICD Gehäuse |
| Variante B: | rechtsventrikulären Ringelektrode - ICD Gehäuse |
| Variante C: | distale Schockwendel - ICD Gehäuse |
| Variante D: | proximale Schockwendel - ICD Gehäuse |

Die bevorzugten Ableitungen sind jedoch Variante D, sofern eine proximale Schockwendel vorhanden ist und Variante C, wenn keine proximale Schockwendel vorhanden ist, da in diesen Ableitungen die Abbildung der Vorhoferregung (P-Welle) am günstigsten ist.

Da mehrere Ableitungen für die Bestimmung der P-Welle möglich sind, ist in dem Ausführungsbeispiel eine Auswahlmatrix vorgesehen, die entweder manuell durch den Anwender programmierbar, oder aber automatisch anhand von Elektrodenimpedanzen und Signalqualität die jeweils günstigste Ableitung für die P-Wellenbestimmung auswählt.

Bei der Ausführung als Herzschrittmacher entfallen die dargestellten Schockwendeln 150 und 160. An deren Stelle erfolgt die Elektrokardiogramm-Ableitung über eine atriale Schrittmacherelektrode (wahlweise Tipelektrode oder Ringelektrode) oder aber über eine flottierende atriale Elektrode, die zusätzlich als Ringelektrode auf der Zuleitung der ventrikulären Elektrode untergebracht ist (A+ bzw. VDD-Prinzip).

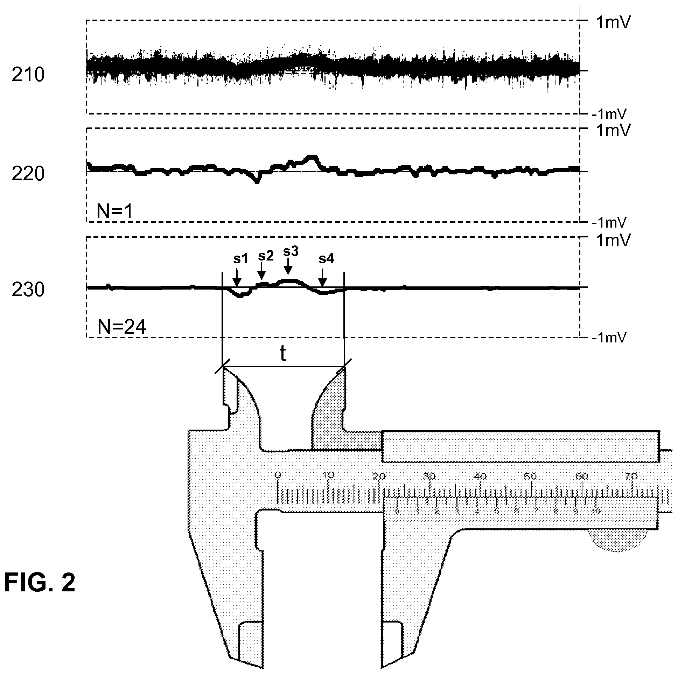

In der Figur 2 sind die Ergebnisse der Signalmittelung (Averaging) zur Erkennung einer P-Welle im Fernfeld-Elektrokardiogramm dargestellt.

Die obere Kurve 210 zeigt ein Eingangssignal mit einer Amplitude von 0,1mV, um eine sehr kleine P-Welle zu simulieren. Zusätzlich ist das Eingangssignal etwas verrauscht, um so die realen Bedingungen der Fernfeld-Elektrokardiogramm-Ableitung zu simulieren.

In der zweiten Kurve 220 wird die Ableitung dieser P-Wellen im Fernfeld-Elektrokardiogramm, abgeleitet mit einem ICD zwischen der proximalen Schockwendel und dem Gehäuse des ICD dargestellt. Die neben der zu erkennenden P-Welle abgebildeten Störsignale lassen noch keine automatische P-Wellenerkennung durch den ICD zu.

In der dritten Kurve 230 ist das Signal dargestellt nachdem es über 24 Herzzyklen gemittelt wurde. Die Signalmittelung wird dabei auf eine jeweilige detektierte atriale Herzaktion (atriales Senseereignis) hin synchronisiert. Die atriale Herzaktion wird in diesem Ausführungsbeispiel in einem Elektrokardiogramm detektiert, das über die proximale Schockelektrode 160 und das Gehäuse des Implantates 110 aufgenommen wurde. Alternativ kann eine separate rechtsatriale Elektrode für das atriale Sensing verwendet werden.

Die Signalqualität nach 24 Mittelungen (Signal 230) ist bereits hinreichend, um eine automatische Erkennung der P-Welle einschließlich ihrer Komponenten (rechtes-linkes Atrium) im ICD realisieren zu können.

Basierend auf dem hinreichend gemittelten Signal 230 ist es nun möglich, Parameter zu bestimmen, die mit der P-Wellen-Dispersion korrelieren. Der dabei wichtigste Parameter stellt die Dauer (t) (auch Breite genannt) der P-Welle dar, die im Implantat bestimmt und gespeichert wird. Die Dauer der gemittelten P-Welle entspricht bei hinreichend gewähltem Ableitungsvektor und Zahl der gemittelten Zyklen der P-Wellen-Dispersion aus dem 12-Kanal-Oberflächen-Elektrokardiogramm.

Eine weitere Verbesserung der diagnostischen Aussage - insbesondere zur Demaskierung mehrere Fokusse der atrialen Erregung - stellt eine Zählung der Unstetigkeitsstellen (S1..S4) in der gemittelten P-Welle 230 dar.

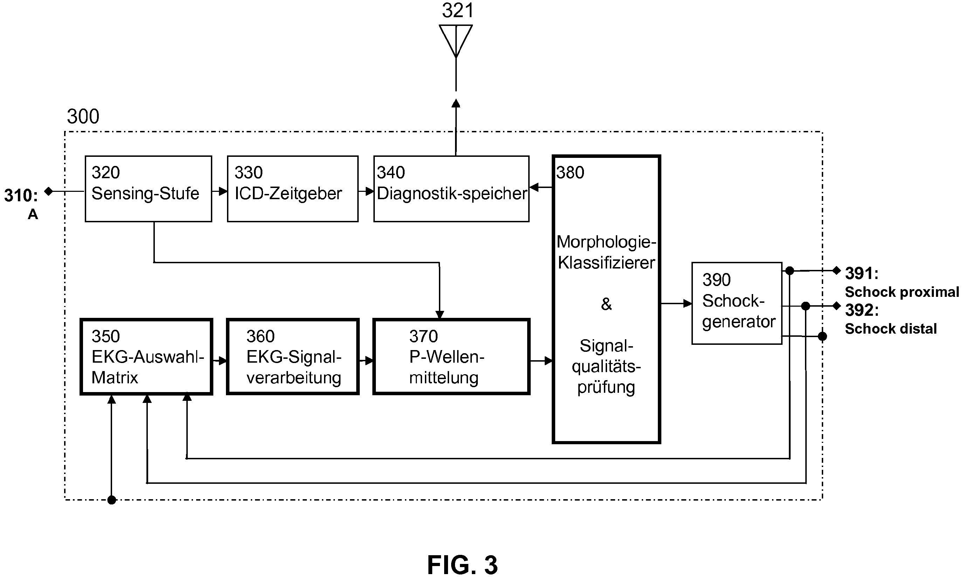

In der Figur 3 sind einige Komponenten eines ICDs, der für eine erfindungsgemäß beschriebene P-Wellen-Dispersionsanalyse ausgelegt ist, in Form eines Blockschaltbilds dargestellt.

Der ICD ist hier zusätzlich mit einer rechtsatrialen Wahrnehmungs- und Stimulationselektrode 310 verbunden. Das mit Hilfe dieser Elektrode aufgenommene (abgeleitete) intrakardiale Elektrokardiogramm wird in einer konventionellen ICD-Sensingstufe 320 analysiert und die detektieren atrialen Herzaktionen (atrialen Ereignisse; P-Sense) werden anschließend einem ICD-Zeitgeber 330 für die Therapiesteuerung und zur Rhythmusdiagnostik 340 zur Verfügung gestellt.

Nicht dargestellt ist der ventrikuläre Wahrnehmungs- und Stimulationskanal, da dieser für die erfindungsgemäße Implementierung keine Änderung gegenüber dem Stand der Technik erfährt.

Die mit einem Schockgenerator 390 des ICD verbundenen Schockelektroden 391 und 392 und das elektrisch leitfähige Gehäuse 300 des ICD sind zusätzlich mit einer Fernfeld-Elektrokardiogramm-Auswahlmatrix 350 verbunden. Diese Auswahlmatrix 350 legt fest, welche der Elektroden für die Ableitung (Aufnahme) eines Fernfeldelektrokardiogramms zur P-Wellen-Dispersionanalyse verwendet werden. Die Auswahl erfolgt dabei entweder manuell durch eine Programmierung durch den Arzt oder aber automatisch durch eine im ICD durchgeführte Signalqualitätsanalyse (380: "Signalqualitätsprüfung"). Dieses Fernfeld-Elektrokardiogramm wird anschließend in einer Elektrokardiogramm-Signalverarbeitungseinheit 360 vorverarbeitet (verstärkt, digitalisiert, gefiltert) und dann einem Mittelwertbildner 370 als Mittelungseinheit zugeführt. Die Fernfeld-Elektrokardio-gramm-Auswahlmatrix 350 und die Elektrokardiogramm-Signalverarbeitungseinheit 360 bilden zusammen eine Fernfeldelektrokardiogrammerfassungseinheit dar. Der mit dieser verbundene Mittelwertbildner 370 führt eine auf eine jeweils detektierte atriale Herzaktion (atriales Ereignis 320) hin getriggerte Signalmittelung durch und stellt diese gemittelten P-Wellenaufzeichnungen einem Morphologieklasifizierer 380 zur Verfügung. Dieser Morphologieklassifizierer 380 vermisst die gemittelte P-Welle hinsichtlich ihrer Signaldauer (Signalbreite), ggf. hinsichtlich der Anzahl ihrer Unstetigkeitsstellen und optional weiterer morphologischer Charakteristika. Diese Information wird dann einem Diagnostikspeicher 340 zur Verfügung gestellt, wobei eine Zuordnung zum atrialen Rhythmus (330->340) erhalten bleibt.

Die im Diagnostikspeicher 340 verfügbare Information zur P-Wellen-Dispersion und zum atrialen Rhythmus kann über eine Nah- oder Fernfeldtelemetrie 321 dem Arzt übermittelt und angezeigt werden.

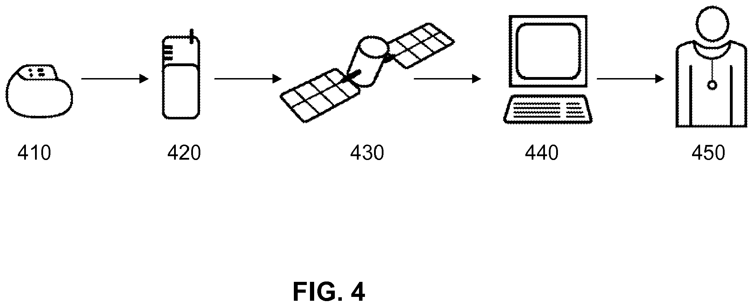

In Figur 4 ist ein erweitertes Gesamtsystem zur kontinuierlichen Überwachung der P-Wellendispersion dargestellt. Hier ist das oben beschriebene elektronische Implantat 410 mit einer Relaisstation oder Basisstation 420 via RF-Telemetrie (bevorzugt im MICS-Band) verbunden und überträgt die Information bezüglich der Freiheit von Vorhofflimmern periodisch an diese Relaisstation 420. Diese wiederum überträgt diese Information via Datenübertragungsnetzwerk 430 an einen Fernüberwachungsserver 440 auf den der Arzt z. B. via Internet Zugriff hat und somit die Möglichkeit besteht, den Patienten telemedizinisch zu betreuen (z. B. Medikamentenmonitoring, Einbestellung zur Ablationstherapie).

Optional kann das Implantat beim erneuten Auftreten von Vorhofflimmern bzw. einer Periode, in der die Kriterien zum Nachweis der Vorhofflimmerfreiheit nicht erfüllt sind, sofort eine Nachricht an das Fernüberwachungssystem senden und der Fernüberwachungsserver versendet dann eine spezielle Nachricht an den betreuenden Arzt (z. B. als SMS, Fax, Email oder eine besonders auffällige Kennzeichnung der Patienten in der Fernnachsorgedatenbank).

Zur korrekten Analyse des klassifizierten Rhythmus durch den Arzt wird zu den als nichtvorhofflimmerfreien Perioden klassifizierten Ereignissen immer auch ein gespeichertes IEGM mit an den Fernüberwachungsserver versendet.

In der Figur 5 ist eine mehrkanalige Bestimmung der P-Wellenbreite ("Dispersion") an einem Beispiel dargestellt. In diesem Beispiel wird eine erste Fernfeld-Elektrokardiogramm-Ableitung 510) aufgezeichnet und gemittelt und simultan eine zweite Fernfeld-Elektrokardiogramm-Ableitung 530 aufgezeichnet und gemittelt. Beide Fernfeld-Elektrokardiogramm-Ableitungen sind so ausgewählt, dass das atriale Elektrokardiogramm gut abgebildet werden kann. Zusätzlich wird auch noch das rechtsventrikulär (alternativ linksventrikulär) abgeleitete intrakardiale Elektrokardiogramm aufgezeichnet. Die Bestimmung der P-Wellendauer erfolgt nun so, dass in den beiden gemittelten atrialen Ableitungen nach dem frühesten Beginn 540 einer atrialen Erregung gesucht wird. Anschließend wird das Ende 550 der atrialen Erregung in den beiden gemittelten atrialen Elektrokardiogrammen bestimmt. Da typischerweise auch die ventrikuläre Erregung in den atrialen Fernfeld-Elektrokardiogramms sichtbar ist, wird zudem das Analysefenster für die atrialen Erregungen nach Hinten begrenzt. Hierfür wird die Information aus dem ventrikulären IEGM genutzt. Das atriale Analysefenster wird bei der frühesten nachweisbaren ventrikulären Erregung 560 begrenzt.

In der Figur 6 ist ein mehrkanaliger Detektor zur Bestimmung der P-Wellenbreite nach dem in Figur 5 illustrierten Verfahren dargestellt. Dieser Detektor ist verbunden mit einer rechtsventrikulären Elektrode (600: RV), Elektroden für eine ersten Fernfeld-Ableitung (630: FF1) und Elektroden für eine zweite Fernfeld-Ableitung (660: FF2). Die Elektroden für die erste Fernfeld Ableitung können z. B. die proximale Schockwendel einer ICD-Elektrode und das ICD-Gehäuse sein. Die Elektroden für die zweite Fernfeld Ableitung können z. B. der proximale Ring einer Coronar-Sinus-Elektrode und das ICD-Gehäuse sein.

Der rechtsventrikuläre Elektrodenanschluss ist mit einer herkömmlichen Sensingstufe 610 zur IEGM-Signalverarbeitung verbunden. Diese wiederum leitet die aufbereiteten IEGM-Signale an einen R-Wellendetektor weiter, der derart ausgeprägt ist, dass dieser mindestens den frühesten Beginn der R-Welle detektiert und an den daran angeschlossenen P-Wellenbreitendetektor 690 weitergibt.

Die beiden Fernfeld-Anschlüsse 630 und 660 sind jeweils mit einer Elektrokardiogramm-Signalverarbeitungseinheit 640 und 670 verbunden. Diese Elektrokardiogramm-Signalverarbeitungseinheiten 640 und 670 sind derart ausgeprägt, dass diese die zu erfassenden atrialen Signale maximal verstärken und breitbandig filtern, sodass die gesamte atriale Erregung erfasst werden kann. Für die Elektrokardiogramm-Signalverarbeitung des ersten Fernfeld-Kanals 640 können dabei andere Parameter eingestellt werden, wie für die Elektrokardiogramm-Signalverarbeitungseinheit des zweiten Fernfeld-Kanals 670. Optional können die Elektrokardiogramm-Signalverarbeitungseinheiten Algorithmen beinhalten, die eine automatische Schlag-zu-Schlag-Anpassung der Parameter durchführt, um so optimal aufgezeichnete Elektrokardiogramm-Signale zu erfassen.

Die Fernfeld-Elektrokardiogramm-Signalverarbeitungseinheiten sind jeweils einer P-Wellenmittelungseinheit 650 und 680 zugeordnet. Diese Einheiten mitteln das artiale Elektrokardiogramm über eine definierte Anzahl von Herzzyklen, wobei diese Anzahl entweder fest voreingestellt wird oder aber durch eine Signalqualitätsprüfung automatisch bestimmt wird (z. B. basierend auf der Auswertung des Signal-Rauschverhältnisses). Die Ergebnisse der P-Wellenmittelung (650, 680) werden ebenfalls dem P-Wellenbreitendetektor (690) zugeführt. Dieser P-Wellenbreitendetektor bestimmt dann gemäß der Beschreibung von Figur 5 die Dauer der P-Welle.

## Patentansprüche

1. Elektromedizinisches Implantat mit
- einer Fernfeldelektrokardiogrammerfassungseinheit (350, 360) zum Aufnehmen eines Fernfeld-Elektrokardiogrammsignals, die mit wenigstens zwei Elektroden zur Aufnahme elektrischer Signals verbunden oder zu verbinden sind, die den Verlauf eines Fernfeld-Elektrokardiogramms des rechten und des linken Vorhofes widerspiegeln,
- mit einer Detektionseinheit (320), die mit der Aufhahmeeinheit verbunden oder Teil derselben ist und ausgebildet ist, für atriale Herzaktionen kennzeichnende Signalmerkmale in einem Fernfeld-Elektrokardiogrammsignal zu detektieren,
- einer Mittelungseinheit (370), die mit der Aufnahmeeinheit und der Detektions-einheit verbunden und ausgebildet ist, ein gemitteltes P-Wellen-Signal zu generieren, indem die Mittelungseinheit eine Mehrzahl von einer jeweiligen detektierten atrialen Herzaktion zugeordneten Signalabschnitten des Elektrokardiogrammsignals miteinander mittelt, und
- einer Auswerteeinheit (380), die mit der Mittelungseinheit (370) verbunden und ausgebildet ist, in dem jeweiligen gemittelten P-Wellen-Signal die Dauer einer gemittelten P-Welle zu bestimmen,
- eine Monitoringeinheit, die mit der Auswerteinheit verbunden ist und die ausgebildet ist, einen der P-Wellen-Dispersion entsprechenden Parameter, nämlich die von der Auswerteeinheit ermittelte Dauer einer gemittelten P-Welle, regelmäßig zu überwachen,
**gekennzeichnet dadurch, dass** die Fernfeldelektrokardiogrammerfassungseinheit dazu ausgebildet ist, wenigstens zwei Fernfeld-Elektrokardiogrammsignale parallel aufzunehmen und jeweils zu mitteln, um die P-Wellendauer zu bestimmen.

2. Implantat gemäß Anspruch 1, **dadurch gekennzeichnet, dass** die Detektionseinheit ausgebildet ist, für atriale Herzaktionen kennzeichnende Signalmerkmale in einem gefilterten Elektrokardiogrammsignal zu detektieren.

3. Implantat gemäß einem der Ansprüche 1 bis 2, **dadurch gekennzeichnet, dass** die Fernfeldelektrokardiogrammerfassungseinheit mit einem elektrisch leitenden Abschnitt eines Gehäuses des Implantats als einer ersten der wenigstens zwei Elektroden verbunden ist.

4. Implantat gemäß Anspruch 3, **dadurch gekennzeichnet, dass** die Fernfeldelektrokardiogrammerfassungseinheit mit einer zur intrakardialen Positionierung geeigneten Defibrillationselektrode als zweiter der wenigstens zwei Elektroden verbunden ist.

5. Implantat gemäß einem der Ansprüche 1 bis 4, **dadurch gekennzeichnet, dass** Auswerteeinheit eine Einheit zum Bestimmen eines Anfangszeitpunkts einer gemittelten P-Welle enthält oder mit einer solchen verbunden ist sowie eine Einheit zum Bestimmen eines Endzeitpunkts einer gemittelten P-Welle enthält oder mit einer solchen verbunden ist und ausgebildet ist, die Dauer einer gemittelten P-Welle als Zeitdifferenz zwischen Anfangszeitpunkt und Endzeitpunkt zu bestimmen.

6. Implantat gemäß Anspruch 5, **dadurch gekennzeichnet, dass** für die Einheit zum Bestimmen eines Anfangszeitpunkts einer gemittelten P-Welle enthält und für die Einheit zum Bestimmen eines Endzeitpunkts einer gemittelten P-Welle jeweils eigene Detektionsparameter vorgebbar sind.

7. Implantat gemäß 5 oder 6, **dadurch gekennzeichnet, dass** die Mittelungseinheit, die Auswerteeinheit oder die Einheit zum Bestimmen eines Endzeitpunkts einer gemittelten P-Welle dazu ausgebildet ist, von einer jeweiligen P-Welle oder einem zu mittelnden Signalabschnitt ein zeitgleiches ventrikuläres Elektrokardiogramm abzuziehen.

8. Implantat gemäß einem der Ansprüche 1 bis 7, **dadurch gekennzeichnet, dass** die Mittelungseinheit ausgebildet ist, die miteinander zu mittelnden Signalabschnitte so miteinander zu mitteln, dass sie über die jeweiligen Zeitpunkte der Detektion einer atrialen Herzaktion eines jeden Signalabschnitts miteinander synchronisiert sind.

9. Implantat gemäß einem der Ansprüche 1 bis 8, **dadurch gekennzeichnet, dass** die Mittelungseinheit ausgebildet ist, eine einstellbare Anzahl von aufeinander folgenden Signalabschnitten miteinander zu mitteln.

10. Implantat gemäß einem der Ansprüche 1 bis 9, **dadurch gekennzeichnet, dass** die Fernfeldelektrokardiogrammerfassungseinheit dazu ausgebildet ist, ein breitbandiges Fernfeld-Elektrokardiogramm über eine rechtsatriale, flottierende Elek-trode aufzunehmen.

## Claims

1. An electromedical implant comprising
- a far-field electrocardiogram capturing unit (350, 360) for recording a far-field electrocardiogram signal, which unit is connected or can be connected to at least two electrodes for recording electrical signals which reflect the course of a far-field electrocardiogram of the right and left atria,
- with a detection unit (320), which is connected to the recording unit or is part thereof and is designed to detect signal features characterising atrial heart actions in a far-field electrocardiogram signal,
- an averaging unit (370), which is connected to the recording unit and the detection unit and is designed to generate an averaged P-wave signal in that the averaging unit averages a plurality of signal portions of the electrocardiogram signal associated with a particular detected atrial heart action with one another, and
- an evaluation unit (380), which is connected to the averaging unit (370) and is designed to determine the duration of an averaged P-wave in a particular averaged P-wave signal,
- a monitoring unit, which is connected to the evaluation unit and which is designed to regularly monitor a parameter corresponding to the P-wave dispersion, specifically the duration of an averaged P-wave determined by the evaluation unit,
**characterised in that**
the far-field electrocardiogram capturing unit is designed to record at least two far-field electrocardiogram signals in parallel and to average them in order to determine the P-wave duration.

2. The implant according to claim 1, **characterised in that** the detection unit is designed to detect signal features characterising atrial heart actions in a filtered electrocardiogram signal.

3. The implant according to either one of claims 1 or 2, **characterised in that** the far-field electrocardiogram capturing unit is connected to an electrically conductive portion of a housing of the implant as a first of the at least two electrodes.

4. The implant according to claim 3, **characterised in that** the far-field electrocardiogram capturing unit is connected to a defibrillation electrode suitable for intracardial positioning as a second of the at least two electrodes.

5. The implant according to any one of claims 1 to 4, **characterised in that** the evaluation unit contains a unit for determining a start time of an averaged P-wave or is connected to such a unit and contains a unit for determining an end time of an averaged P-wave or is connected to such a unit and is designed to determine the duration of an averaged P-wave as a time difference between the start time and end time.

6. The implant according to claim 5, **characterised in that** separate detection parameters can be specified for the unit for determining a start time of an averaged P-wave and for the unit for determining an end time of an averaged P-wave.

7. The implant according to claim 5 or 6, **characterised in that** the averaging unit, the evaluation unit or the unit for determining an end time of an averaged P-wave is designed to deduct an isochronous ventricular electrocardiogram from a particular P-wave or a signal portion to be averaged.

8. The implant according to any one of claims 1 to 7, **characterised in that** the averaging unit is designed to average with one another the signal portions that are to be averaged with one another such that they are synchronised with one another via the various times of the detection of an atrial heart action of each signal portion.

9. The implant according to any one of claims 1 to 8, **characterised in that** the averaging unit is designed to average with one another an adjustable number of successive signal portions.

10. The implant according to any one of claims 1 to 9, **characterised in that** the far-field electrocardiogram capturing unit is designed to record a broadband far-field electrocardiogram via a right-atrial, floating electrode.

## Revendications

1. Implant électromédical doté
- d'une unité d'enregistrement d'un électrocardiogramme en champ lointain (350, 360) pour l'enregistrement d'un signal d'électrocardiogramme en champ lointain, qui est reliée ou est à relier avec au moins deux électrodes pour l'enregistrement de signaux électriques qui reflètent le déroulement d'un électrocardiogramme en champ lointain de l'oreillette droite et de l'oreillette gauche,
- doté d'une unité de détection (320) qui est reliée avec l'unité d'enregistrement ou constitue une partie de celle-ci et est conçue pour détecter des particularités de signaux caractérisant des actions cardiaques atriales dans un signal d'électrocardiogramme en champ lointain,
- une unité de signalement (370) qui est reliée avec l'unité d'enregistrement et l'unité de détection et est conçue pour générer un signal d'ondes P moyenné par le fait que l'unité de signalement effectue la moyenne d'une pluralité de segments de signaux du signal d'électrocardiogramme associés à une action cardiaque atriale détectée respective et
- une unité d'exploitation (380) qui est reliée avec l'unité de signalement (370) et est conçue pour déterminer la durée d'une onde P moyennée dans le signal d'onde P moyenné respectif,
- une unité de surveillance qui est reliée avec l'unité d'exploitation et est conçue pour surveiller de manière régulière un paramètre correspondant à la dispersion des ondes P, à savoir la durée d'une onde P moyennée déterminée par l'unité d'exploitation,
**caractérisé en ce que**
l'unité de détection d'électrocardiogramme en champ lointain est conçue pour enregistrer parallèlement au moins deux signaux d'électrocardiogramme en champ lointain et les moyenner respectivement afin de déterminer la durée d'onde P.

2. Implant selon la revendication 1, **caractérisé en ce que** l'unité de détection est conçue pour détecter des particularités de signal caractérisant des actions cardiaques atriales dans un signal d'électrocardiogramme filtré.

3. Implant selon l'une des revendications 1 à 2, **caractérisé en ce que** l'unité de capture d'électrocardiogramme en champ lointain est reliée avec un segment électriquement conducteur d'un boîtier de l'implant sous la forme d'une première électrode parmi les au moins deux électrodes.

4. Implant selon la revendication 3, **caractérisé en ce que** l'unité de capture d'électrocardiogramme en champ lointain est reliée avec une électrode défibrillation appropriée pour le positionnement intracardiaque sous la forme de la deuxième électrode parmi les au moins deux électrodes.

5. Implant selon l'une des revendications 1 à 4, **caractérisé en ce que** l'unité d'exploitation contient une unité pour la détermination d'un instant de départ d'une l'onde P moyennée, ou est reliée avec une telle unité, ainsi qu'une unité pour la détermination d'un instant de fin d'une onde P moyennée, ou est reliée avec une telle unité, et est conçue pour déterminer la durée d'une onde P moyennée sous forme d'une différence de temps entre un instant de départ et un instant de fin.

6. Implant selon la revendication 5, **caractérisé en ce que** des paramètres de détection propres respectifs peuvent être fixés pour l'unité pour la détermination d'un instant de départ d'une onde P moyennée et pour l'unité pour la détermination d'un instant de fin d'une onde P moyennée.

7. Implant selon la revendication 5 ou la revendication 6, **caractérisé en ce que** l'unité de signalement, l'unité d'exploitation, ou l'unité destinée à la détermination d'un instant de fin d'une onde P moyennée, est conçue pour enlever un électrocardiogramme ventriculaire de même durée d'une onde P respective ou d'un segment de signal à moyenner.

8. Implant selon l'une des revendications 1 à 7, **caractérisé en ce que** l'unité de signalement est conçue pour effectuer la moyenne de segments de signal à moyenner de telle manière qu'ils soient synchronisés ensemble par le biais des instants respectifs de la détection d'une action cardiaque atriale dans chacun des segments de signal.

9. Implant selon l'une des revendications 1 à 8, **caractérisé en ce que** l'unité de signalement est conçue pour faire la moyenne d'un nombre réglable de segments de signal se succédant les uns aux autres.

10. Implant selon l'une des revendications 1 à 9, **caractérisé en ce que** l'unité de capture d'électrocardiogramme en champ lointain est conçue pour enregistrer un électrocardiogramme en champ lointain à bande large par le biais d'une électrode atriale droite flottante.
